(19) Europäisches Patentamt
European Patent Office
Office européen des brevets

(11) Veröffentlichungsnummer : **0 487 903 B1**

(12) # EUROPÄISCHE PATENTSCHRIFT

(45) Veröffentlichungstag der Patentschrift : **14.04.93 Patentblatt 93/15**

(51) Int. Cl.$^5$ : **C07C 29/04,** C07C 29/14, C07C 31/20, C07C 29/141, C07C 45/64

(21) Anmeldenummer : **91118212.9**

(22) Anmeldetag : **25.10.91**

(54) **Verfahren zur Herstellung von 1,3-Propandiol.**

(30) Priorität : **30.11.90 DE 4038192**

(43) Veröffentlichungstag der Anmeldung : **03.06.92 Patentblatt 92/23**

(45) Bekanntmachung des Hinweises auf die Patenterteilung : **14.04.93 Patentblatt 93/15**

(84) Benannte Vertragsstaaten : **AT BE CH DE DK ES FR GB IT LI LU NL SE**

(56) Entgegenhaltungen :
**EP-A- 0 352 949**
**EP-A- 0 412 337**
**DE-A- 2 057 399**
**US-A- 4 031 038**

(56) Entgegenhaltungen :
**US-A- 4 094 914**
**CHEMICAL ABSTRACTS, vol. 104, no. 25, 23. Juni 1986, Columbus, Ohio, US; abstract no. 224589C, HIRAI, HIDEFUMI ET AL.: "Colloidal palladium supported on chelate resin containing iminodiacetic acid groups as hydrogenation catalyst" Seite 585, Spalte 1**

(73) Patentinhaber : **Degussa Aktiengesellschaft
Weissfrauenstrasse 9
W-6000 Frankfurt am Main 1 (DE)**

(72) Erfinder : **Arntz, Dietrich, Dr.
Lorsbacherstrasse 32
W-6370 Oberursel (DE)**
Erfinder : **Wiegand, Norbert, Dr.
Birkenhainer Strasse 15a
W-6450 Hanau (DE)**

**EP 0 487 903 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von 1,3-Propandiol durch Hydratisierung von Acrolein in Gegenwart eines chelatbildenden Ionenaustauscherharzes mit nachfolgender katalytischer Hydrierung des 3-Hydroxypropionaldehyds.

1,3-Propandiol besitzt als Monomerbaustein für Polyester und Polyurethane sowie als Ausgangsstoff für die Synthese cyclischer Verbindungen vielseitige Anwendungsmöglichkeiten.

Wie aus der US-PS 2,434,110 bekannt ist, läßt sich Acrolein in Gegenwart eines sauren Katalysators hydratisieren, wobei 3-Hydroxypropionaldehyd gebildet wird. Die Umsetzung erfolgt vorzugsweise bei erhöhter Temperatur unter Verwendung einer 5 bis 30 gew.-%igen Lösung von Acrolein in Wasser und einer Säure, wie z. B. Schwefelsäure, Phosphorsäure oder sauren Salzen dieser Säuren, als Katalysator. Das bei der Hydratisierung erhaltene Reaktionsgemisch wird, ggf. nach Entfernung von nicht umgesetztem Acrolein, in Gegenwart üblicher Hydrierkatalysatoren hydriert. Für die Hydrierung des 3-Hydroxypropionaldehyds zu 1,3-Propandiol sind Katalysatoren, welche ein oder mehrere hydrierwirksame Metalle, wie z. B. Fe, Co, Ni, Cu, Ag, Mo, W, V, Cr, Rh, Pd, Os, Ir, Pt enthalten, geeignet.

Nachteilig an dem Verfahren der US-PS 2,434,110 sind die niedrigen Ausbeuten an 1,3-Propandiol, welche insbesondere auf Acrolein verbrauchende Nebenreaktionen während der Hydratisierungsstufe zurückzuführen sind. Die Selektivität der mineralsauer katalysierten Hydratisierung ist ferner stark vom Acrolein-Umsatz abhängig. Um eine akzeptable Selektivität zu erzielen, beendet man die Hydratisierung bei niedrigem Acrolein-Umsatz, mit dem Nachteil einer geringen Raum-Zeit-Ausbeute.

Es hat nicht an Versuchen gefehlt, die Nachteile des zuvor gewürdigten Verfahrens zu mindern. Beispielsweise werden durch Anlagern von niederen Carbonsäuren an Acrolein durch Erhitzen (US-PS 2,638,479) oder in Gegenwart eines basischen Ionenaustauscherharzes Carbonsäureester des 3-Hydroxypropionaldehyds erhalten, die zu den entsprechenden Estern des 1,3-Propandiols hydriert werden können. Nachteilig sind bei diesen Verfahren die zusätzlich notwendigen Schritte zur Verseifung des Esters und zur Recyclierung der Carbonsäure sowie die unerwünschte Bildung von n-Propanol und seinen Carbonsäureestern bei der Hydrierung (DE-OS 20 57 399). Bekannt ist auch die Hydratisierung von Acrolein mit Kohlendioxid als Katalysator, jedoch erfordert dieses Verfahren lange Reaktionszeiten - vgl. DE-OS 19 05 823.

Es wurde festgestellt, daß unter Verwendung von z. B. Phosphorsäure oder Dihydrogenphosphaten als Katalysator zwar die Hydratisierung von Acrolein durchgeführt werden kann, bei der anschließenden Hydrierung aber Probleme auftreten, wenn die so erhaltene Hydroxypropionaldehyd-Lösung ohne sorgfältige Abtrennung des Hydratisierungskatalysators hydriert wird:

Bei Verwendung von an sich sehr wirksamen Nickel-Hydrierkatalysatoren wird bei wiederholtem Einsatz des Katalysators dieser schneller desaktiviert, als wenn ein säure- und salzfreies Reaktionsgemisch hydriert wird. Dies führt zu einem erhöhten Katalysatorverbrauch. Zusätzlich kommt es durch die Anwesenheit des Hydratisierungskatalysators während der destillativen Aufarbeitung zu Produktverlusten durch Zersetzung bzw. im Falle einer vorangehenden Neutralisation zu Verstopfungen und Verkrustungen in der Anlage. Auch ist die Beseitigung von Destillationsrückständen bei einem Gehalt an anorganischen Salzen schwieriger und damit teurer als ohne diese Salze.

Die aufgezeigten Probleme lassen sich zum Teil umgehen, wenn man den Hydratisierungskatalysator mittels Ionenaustauschern vor der Hydrierung aus dem Reaktionsgemisch entfernt oder 3-Hydroxypropionaldehyd extraktiv aus dem Reaktionsgemisch abtrennt und dann hydriert. Beide alternativen Maßnahmen zur Reduzierung des Verbrauchs an teurem Hydrierkatalysator machen aber zusätzliche Einrichtungen erforderlich, führen zu einem höheren Energieaufwand und zu Abwasserproblemen und erhöhen somit die Herstellkosten für 1,3-Propandiol.

Gemäß dem Verfahren der US-PS 3,536,763 wird die Hydratisierung von Acrolein in Gegenwart schwachsaurer Kationenaustauscherharze, deren funktionelle Gruppen nur Carboxylgruppen sind, bei 40 bis 120 °C durchgeführt. Vorzugsweise sollen 0,1 bis 5 % der funktionellen Gruppen in Form eines Alkali-, Erdalkali- oder Erdmetallcarboxylats vorliegen. Die Ausbeuten an 3-Hydroxypropionaldehyd werden mit etwa 80 % angegeben, wobei die Ausbeuten vom Acrolein-Umsatz im Bereich von 25 bis 65 % praktisch nicht abhängig sein sollen. Dieses Dokument umfaßt auch die an sich bekannte Hydrierung des 3-Hydroxypropionaldehyds zu 1,3-Propandiol.

Bei der Nacharbeitung des Verfahrens der US-PS 3,536,763 konnte zwar die katalytische Wirksamkeit der Ionenaustauscherharze mit Carboxylgruppen bestätigt werden, der Grad der Wirksamkeit ließ aber die Verwendung dieser Ionenaustauscher in technischen Anlagen als nicht geeignet erscheinen. Es zeigte sich nämlich, daß diese Katalysatoren höhere Temperaturen und längere Reaktionszeiten erfordern, was der erwünschten hohen Raum-Zeit-Ausbeute und hohen Selektivität zuwiderläuft.

Aufgabe der vorliegenden Erfindung ist somit, ein verbessertes Verfahren zur Herstellung von 1,3-Propandiol durch Hydratisierung von Acrolein in Gegenwart von Ionenaustauscherharzen mit nachfolgender katalytischer Hydrierung zur Verfügung zu stellen, das in der Hydratisierungsstufe mit guter Selektivität und möglichst hoher Raum-Zeit-Ausbeute durchgeführt werden kann. Das bei Hydratisierung erhaltene Reaktionsgemisch sollte auch den Hydrierkatalysator möglichst wenig desaktivieren, um die Wiederverwendung des Hydrierkatalysators in Folgechargen zu ermöglichen beziehungsweise die Standzeit eines Festbett-Hydrierkatalysators zu verlängern und damit die Wirtschaftlichkeit des Verfahrens zu verbessern.

Das erfindungsgemäße Verfahren zur Herstellung von 1,3-Propandiol durch Hydratisierung von Acrolein in Gegenwart eines Ionenaustauschers unter Bildung von 3-Hydroxypropionaldehyd, wobei Acrolein und Wasser im Gewichtsverhältnis von 1:2 bis 1:20 bei 30 bis 120 °C und einem Druck im Bereich von 1 bis 20 bar umgesetzt werden, Abtrennung des Ionenaustauschers und, soweit vorhanden, des nicht umgesetzten Acroleins aus dem Reaktionsgemisch und anschließende katalytische Hydrierung des 3-Hydroxypropionaldehyds unter an sich bekannten Bedingungen in flüssiger oder gasförmiger Phase unter Verwendung üblicher Hydrierkatalysatoren, ist dadurch gekennzeichnet, daß man chelatbildende Ionenaustauscher verwendet, welche an die Polymermatrix eines Polymerisationsharzes gebundene Ankergruppen der allgemeinen Formel

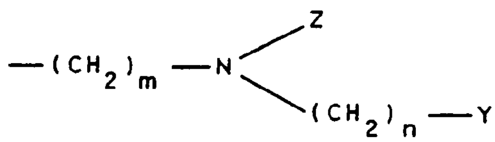

enthalten, worin bedeuten

Z: H, $C_1$- bis $C_6$-Alkyl, $-CH_2-CH(CH_3)-Y'$ oder $-(CH_2)_o-Y'$;

Y und Y': gleich oder verschieden;
-COOH, -OH, Pyridyl oder $-P(O)(CH_2OH)OH$, wobei die sauren funktionellen Gruppen teilweise in Form ihrer Alkali-, Erdalkali- oder Erdmetallsalze vorliegen können.

m: 0, 1, 2 oder 3

n: 1, 2 oder 3 für Y = -COOH, Pyridyl oder $-P(O)(CH_2OH)OH$;
2 oder 3 für Y = -OH

o: 1, 2 oder 3 für Y' = -COOH, Pyridyl oder $-P(O)(CH_2OH)OH$;
0, 2 oder 3 für Y' = -OH .

Chelatbildende Ionenaustauscher sind lange bekannt. Über die Struktur, Herstellung, Eigenschaften und Verwendung solcher Ionenaustauscher vermittelt das Buch "Chelatbildende Ionenaustauscher" von R. Hering, Akademie Verlag Berlin (1967) einen Überblick.

Die Polymermatrix (loc. cit., Seiten 25-58) der Polymerisationsharze basiert vorzugsweise auf einem Copolymerisat aus Styrol und Divinylbenzol, kann aber auch ein Acrylpolymeres oder Copolymeres aus Acrylverbindungen und Allylverbindungen sowie ein Polymeres eines funktionalisierten Epoxids sein. Die Ankergruppen können in die Polymerisationsharze in an sich bekannter Weise in die Polymermatrix eingeführt werden, etwa (a) durch Chlormethylierung des Polymers mit nachfolgender Umsetzung mit z. B. Glycin, Sarkosin, Iminodiessigsäure oder Iminodipropionsäure, Ethanolamin, Diethanolamin oder Ethanolaminmonoessigsäure oder (b) durch Nitrierung, Reduktion und Umsetzung z. B. mit Chloressigsäure. In einzelnen Fällen kann ein die Ankergruppe enthaltendes polymerisierbares Monomeres mit copolymerisationsfähigen anderen Monomeren gemeinsam polymerisiert werden - beispielhaft wird auf die Copolymerisation von N-Allyliminodipropionsäure mit Acrylnitril verwiesen. Ionenaustauscher, in welchen Y und/oder Y' die Gruppe $-(O)P(OH)CH_2OH$ bedeutet, sind aus der EP-A 352 949 (GB 017051 v. 18.08.88) bekannt. Ionenaustauscher, die Picolylamingruppen enthalten, sind aus der US-PS 4,031,038 bekannt.

Bevorzugt werden makroporöse Polymerisationsharze auf der Basis von Styrol/Divinylbenzol-Copolymeren verwendet, wobei die Aminogruppe vorzugsweise über eine Methylengruppe - m ist hier also 1 - an die aromatische Matrix gebunden ist. Besonders geeignete chelatbildende Ionenaustauscher weisen als Ankergruppe die Methyleniminodiessigsäuregruppe auf - n, m, o = 1 und Z = $CH_2COOH$ und Y = COOH, wobei bekannt ist, daß ein gewisser Anteil der Ankergruppen aus Aminoessigsäurefunktionen bestehen kann, d. h. m, n = 1, Z = H und Y = -COOH.

Die chelatbildenden Ionenaustauscher mit mindestens einer Säuregruppe in der Ankergruppe können in Form der freien Säure (H-Form) eingesetzt werden; ein Teil der Säuregruppen - insbesondere bis zu einem Drittel der totalen Austauschkapazität des Harzes - kann auch in Form eines Alkali-, Erdalkali- oder Erdmetallsalzes vorliegen, Ionenaustauscher, deren Säuregruppen im wesentlichen in der H-Form vorliegen, werden besonders bevorzugt.

Die Überführung eines in der Na-Form gelieferten Austauschers in die H-Form erfolgt in der für Ionenaustauscher üblichen Weise. Die Einstellung eines bestimmten Beladungszustandes mit Metallkationen kann aus der H-Form durch Zugabe der entsprechenden Menge Metallhydroxid, gelöst oder suspendiert, in einer Suspension des Harzes in Wasser erfolgen; ein partiell in der H- und Na-Form vorliegender Austauscher läßt sich aber auch durch Zugabe einer für einen vollständigen Austausch der Na-Ionen nicht ausreichenden Menge an Säure zur Na-Form des Austauscherharzes erhalten. In beiden Fällen wird das Ionenaustauscherharz vor seiner Verwendung durch Waschen mit deionisiertem Wasser von löslichen Salzen und anderen löslichen Bestandteilen befreit. Die Austauschkapazität kann in weiten Bereichen liegen, jedoch haben sich Austauscher mit einer Kapazität im Bereich von etwa 1 bis 3 Äquivalente (H-Form) pro l Austauscherharz bewährt. Die Kapazität ist ein Maß für die Dichte der chelatbildenden Ankergruppen im Austauscherharz.

Unter den Ionenaustauschern, in welchen Y und/oder Y′ die Pyridylgruppe bedeutet, sind solche mit Ankergruppen der Formel

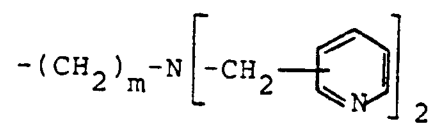

oder

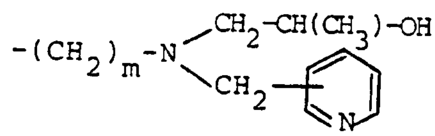

worin m 0, 1, 2 oder 3, insbesondere 1 bedeutet, bevorzugt.

Die erfindungsgemäß zu verwendenden Ionenaustauscher zeichnen sich im allgemeinen durch eine hohe Standzeit aus. Um die höchste Selektivität und damit höchst mögliche Ausbeute an 3-Hydroxypropionaldehyd zu erhalten und über eine lange Betriebszeit aufrechtzuerhalten, ist es empfehlenswert, nicht den höchst möglichen Acrolein-Umsatz anzustreben.

Die Hydratisierung von Acrolein läßt sich, wie aus der zum Zeitpunkt der Priorität der vorliegenden Anmeldung unveröffentlichten Patentanmeldung P 39 26 136.0 hervorgeht, durch Verwendung von Kationenaustauschern mit Phosphonsäuregruppen, insbesondere chelatbildenden Aminophosphonsäuregruppen durchführen. Nach den Ausführungen in der genannten Anmeldung P 39 26 136.0, die am 13.02.91 als EP-A-412 337 veröffentlicht wurde, und im Hinblick auf die carboxylgruppenhaltigen Ionenaustauscher gemäß US-PS 3,536,763 erhaltenen unbefriedigenden Ergebnisse konnte nicht erwartet werden, daß die erfindungsgemäß bevorzugt zu verwendenden carboxylgruppenhaltigen chelatbildenden Kationenaustauscher als Katalysator für die Hydratisierung von Acrolein hervorragend geeignet sind. Unvorhersehbar war ferner die Verwendbarkeit chelatbildender Anionenaustauscher als Hydratisierungskatalysator.

Acrolein und Wasser werden in einem Gewichtsverhältnis von 1:2 bis 1:20, insbesondere 1:3 bis 1:10 und bevorzugt 1:3 bis 1:6, der Hydratisierungsstufe zugeführt. Die Umsetzung zum 3-Hydroxypropionaldehyd erfolgt im Temperaturbereich von 30 bis 120 °C. Eine Temperatur im Bereich von 40 bis 90 °C wird bevorzugt; eine Temperatur unter 40 °C führt im allgemeinen zu langen Reaktionszeiten, eine Temperatur oberhalb 90 °C zu einer verminderten Selektivität und Problemen bezüglich der Standzeit der Austauscherharze. Besonders bevorzugt erfolgt die Hydratisierung bei 50 bis 80 °C.

Im Temperaturbereich unter dem Siedepunkt des Acroleins kann die Umsetzung bei Normaldruck oder mäßigem Druck erfolgen. Bei Umsetzungstemperaturen um oder über dem Siedepunkt von Acrolein wird man unter einem Druck im Bereich von etwa 2 bis 20 bar arbeiten. Im bevorzugten Temperaturbereich von 40 bis 90 °C hat sich ein Druck im Bereich von 2 bis 5 bar als geeignet erwiesen.

Die Hydratisierung wird im allgemeinen bis zu einem Acroleinumsatz im Bereich von 30 bis 90 % oder darüber durchgeführt; ein Umsatz von 40 bis 90 % und insbesondere 50 bis 80 % wird bevorzugt.

Zweckmäßigerweise werden dem Acrolein-Wasser-Gemisch Polymerisationsinhibitoren, wie z. B. Hydrochinon, Hydrochinonmonomethylether oder butylierte Phenole, in wirksamer Menge zugegeben.

Die Hydratisierung kann diskontinuierlich oder kontinuierlich erfolgen, wobei an sich bekannte Reaktoren, wie beispielsweise Rührreaktoren, Schlaufenreaktoren, Schwebebett-, Wirbelbett- und Festbettreaktoren, einsetzbar sind. Zuletzt genannte Reaktoren werden gegenüber Schlaufen- und Rührreaktoren bevorzugt. Die Durchflußgeschwindigkeit durch einen Festbettreaktor, der den chelatbildenden Ionenaustauscher enthält und mit einem heizbaren Mantel versehen ist, sowie die Reaktionstemperatur wird man vorzugsweise so aufeinander abstimmen, daß durch einmalige Passage des Reaktionsgemisches durch den Reaktor der gewünschte Acrolein-Umsatz erreicht wird.

Nach der Abtrennung des Ionenaustauschers, was üblicherweise durch Sedimentation oder Filtration erfolgt oder sich bei Verwendung eines Harzbettes (wie es beispielsweise in der Wasseraufbereitung üblich ist) von allein ergibt, wird das Reaktionsgemisch, soweit erforderlich, von nicht-umgesetztem Acrolein befreit. Die Abtrennung des Acroleins kann in bekannter Weise, insbesondere destillativ, vorzugsweise unter vermindertem Druck und Temperaturen unter 80 °C, verwirklicht werden. Das zurückgewonnene Acrolein kann wieder - nach erfolgter Stabilisierung - in den Prozeß eingespeist werden. Die erhaltene, praktisch acroleinfreie Hydroxypropionaldehydlösung kann vor der Hydrierung z. B. über einen Dünnschichtverdampfer nochmals aufkonzentriert werden.

Die katalytische Hydrierung des 3-Hydroxypropionaldehyds in flüssiger Phase wird in an sich bekannter Weise und in üblichen Hydrierapparaturen durchgeführt. Der Katalysator kann sowohl in suspendierter Form per se oder trägergebunden zur Anwendung gelangen oder sich in einem Festbettreaktor befinden; auch homogene Katalysatoren können herangezogen werden. Als Suspensionskatalysatoren sind Raney-Nickel, das mit verschiedenen anderen Metallen dotiert sein kann, sowie feinverteiltes Platin auf einem Trägermaterial wie z. B. Aktivkohle besonders geeignet. Unter den Festbettkatalysatoren kommen die bei der Würdigung der US-PS 2,434,110 genannten Stoffe zur Anwendung; Nickelkatalysatoren haben sich als besonders wirkungsvolle Katalysatoren erwiesen. Zur Erzielung einer hohen Umsatzrate wird die Hydrierung unter Druck und bei erhöhter Temperatur durchgeführt, wobei die wäßrige Lösung einen pH-Wert im Bereich von 3,0 bis 8,5, worzugsweise um 6, aufweist. Drucke von 20 bis 250 bar, insbesondere 40 bis 140 bar, und Temperaturen von 40 bis 140 °C, insbesondere 60 bis 100 °C, werden bevorzugt.

Prinzipiell kann 3-Hydroxypropionaldehyd auch in der Gasphase katalytisch hydriert werden - vgl. DE-PS 20 54 601, so daß sich an die erfindungsgemäße Hydratisierung auch diese Ausführungsform der Hydrierung anschließen kann.

Wie aus den Beispielen hervorgeht, gelingt es, durch die Verwendung der erfindungsgemäßen chelatbildenden Ionenaustauscher als Hydratisierungskatalysator eine sehr hohe Selektivität bei gleichzeitig hohem Acrolein-Umsatz zu erzielen. Hiermit ist die Hydratisierung mit hoher Raum-Zeit-Ausbeute durchführbar. Die vorbekannten schwachsauren carboxylgruppenhaltigen Ionenaustauscher führen wegen ihrer geringeren Aktivität und geringeren Selektivität zu viel niedrigeren Raum-Zeit-Ausbeuten. Die erfindungsgemäße Hydratisierung führt zu Reaktionsgemischen, welche sich problemlos von nicht umgesetztem Acrolein befreien und dann hydrieren lassen.

**Beispiele 1 bis 5 sowie Vergleichsbeispiele 1 bis 3**

Zur Bestimmung der Aktivität der Ionenaustauscher nach dem Stand der Technik (Vergleichsbeispiele VB1 bis VB3) sowie der Erfindung (Beispiele 1 bis 5) wird folgender Test durchgeführt:

Ein 50 ml-Septumfläschchen wird mit 25 ml Ionenaustauscher-Katalysator befüllt und soviel Wasser zugegeben, daß der Katalysator gerade bedeckt ist. Zusätzlich werden 16 g Wasser und 7,56 g Acrolein (Ac) zugegeben. Die Mischung wird 3 Minuten bei Raumtemperatur durch rotierende Bewegung geschüttelt und die aktuelle Acrolein-Konzentration einer Probe gaschromatographisch bestimmt. Die Probe wird für 60 Minuten im Wasserbad bei der angegebenen Temperatur weitergerührt und analysiert. Der Tabelle 1 sind der Umsatz und die Selektivität nach 1 Stunde Reaktion, die Startkonzentration an Acrolein, die Reaktionstemperatur zu entnehmen.

Ein stark saurer Ionenaustauscher (VB1) führt zu einem hohen Acrolein-Umsatz, jedoch zu einer völlig unzureichenden Selektivität. Mit dem schwachsauren nicht-chelatbildenden Ionenaustauscher (VB2) wird bei mittlerem Umsatz nur eine mittlere Selektivität erhalten. Ein schwach saurer, $Ca^{2+}$-dotierter Ionenaustauscher ist reaktiver, aber ebenfalls nur mittelmäßig selektiv (VB3). Die beispielgemäßen chelatbildenden Ionenaustauscher mit Iminodiessigsäuregruppen (Beispiele 1 bis 3) sowie die chelatbildenden basischen Ionenaustauscher der Beispiele 4 und 5 sind sowohl sehr aktiv als auch sehr selektiv.

Tabelle 1

| | Katalysator Bezeichnung | funktionelle Gruppierung | Ac-Start konz. (Gew.-%) | Temperatur °C | 1 Stunde Umsatz (%) | 1 Stunde Selekt. (%) |
|---|---|---|---|---|---|---|
| VB 1 | ®Lewatit S 100 * | $-SO_3H$ | 17,9 | 70 | 92,0 | 22 |
| VB 2 | ®Lewatit CNP 80 * H-Form | -COOH | 15,1 | 70 | 21,6 | 49 |
| VB 3 | ®Lewatit CNP LF, * H-Form, $Ca^{2+}$-dotiert | -COOH ($Ca^{2+}$) | 15,7 | 70 | 43,4 | 50 |
| Beispiel 1 | ®Amberlite ** IRC-718, H-Form | Iminodiessigsäure | 16,2 | 70 | 77,5 | 82 |
| Beispiel 2 | ®Duolite ES 466 ** H-Form | Iminodiessigsäure | 18,4 | 70 | 71,7 | 77 |
| Beispiel 3 | ®Lewatit TP 208 * H-Form | Iminodiessigsäure (a) | 18,3 | 50 | 70,7 | 87 |
| | | (b) | 18,0 | 40 | 38,5 | 91 |
| Beispiel 4 | ®DOWEX XFS *** 4195.02 | Bispicolylamin | 17,5 | 50 | 36,0 | 74 |
| Beispiel 5 | ®DOWEX XFS *** 43084.00 | Hydroxypropyl-picolylamin | 16,5 | 50 | 39,6 | 84 |

* Firma Bayer AG; ** Firma Rohm & Haas Co; *** Firma Dow Chemical

**Beispiele 6 bis 9**

Die Hydratisierung erfolgte gemäß den vorhergehenden Beispielen. Zum Einsatz kam der Ionenaustauscher des Beispiels 3 (Lewatit TP 208 H-Form), der durch Behandlung mit wäßriger NaOH- (Beispiele 6 und 7), $MgSO_4$- (Beispiel 8) oder $Al(NO_3)_3$-Lösung (Beispiel 9) teilweise mit Metallionen dotiert wurde. Der Dotierungsgrad wurde durch Analyse der im Vakuum getrockneten Harzproben bestimmt. Die Bedingungen und Ergebnisse folgen aus der Tabelle 2;
Reaktionstemperatur 50 °C. Es zeigte sich, daß die teilweise Metallionen-dotierten Ionenaustauscher ohne Selektivitätsabfall bis zu sehr hohen Umsätzen verwendbar sind.

Tabelle 2

| Beispiel | Dotierung Gew.-% | Ac-Konz. Gew.-% | Umsatz nach 4 Stunden (%) | Selektivität nach 4 Stunden (%) |
|---|---|---|---|---|
| 6 | 0,53 Na | 17,7 | 90,5 | 82,8 |
| 7 | 4,2 Na | 17,2 | 89,1 | 85,1 |
| 8 | 0,06 Mg | 17,7 | 89,3 | 80,4 |
| 9 | 0,3 Al | 17,8 | 88,9 | 81,1 |

**Beispiel 10**

In einer Laborapparatur mit einem Festbettreaktor mit Ionenaustauscherharz als Katalysator wird die Hydratisierung von Acrolein kontinuierlich über einen längeren Zeitraum durchgeführt. Umsatz und Selektivität werden durch Analyse der Produktlösung bestimmt.

Die Apparatur besteht aus einem skalierten, kühlbaren 2 l-Glasgefäß für die wäßrige Acrolein-Ausgangslösung, einer HPLC-Pumpe für die Förderung der Reaktionsmischung, einem thermostatierten, druckbeständigen, präzisionsgeschliffenen Glasrohr (300 mm x 26 mm i.D.), das die Ionenaustauscherfüllung aufnimmt und an beiden Enden mit verstellbaren Verschraubungen verschlossen ist, einem Druckhalteventil, sowie einer auf +5 °C gekühlten 2 l-Glasvorlage für die Produktlösung und den notwendigen Thermostatisiereinrichtungen.

Die Acrolein-Wasser-Mischung wird auf +5 °C gekühlt vorgelegt und mittels der HPLC-Pumpe durch das auf Reaktionstemperatur gehaltene Festbett aus 138 ml Ionenaustauscherharz bei konstantem Volumenstrom gepumpt. Nach einer Einfahrzeit von 3-5 h zur Einstellung des stationären Zustands wird nach Wechseln der Vorlage unter konstanten Versuchsbedingungen über einen Zeitraum von mehreren Stunden Acroleinlösungen durch das thermostatisierte Glasrohr gepumpt. Diese Messung wird mehrmals wiederholt. Die jeweils erhaltenen Produktlösungen werden gaschromatographisch analysiert - siehe Tabelle 3.

| | |
|---|---|
| Eingesetzter Ionenaustauscher: | ®Lewatit TP 208 (Bayer AG) H-Form |
| Acrolein-Startkonzentration: | 16,9 Gew.-% |
| Reaktionstemperatur: | 60 °C |
| LHSV (=liquid hourly space velocity): | 0,54 $Std^{-1}$ |

Tabelle 3

| Betriebsdauer (Stunden) | Umsatz % | Selektivität % |
|---|---|---|
| 40 | 86 | 78 |
| 80 | 82 | 82 |
| 120 | 78 | 80 |

**Beispiel 11**

Die Hydratisierung des Acroleins erfolgt in der in Beispiel 10 beschriebenen Weise. Zum Einsatz kam der Ionenaustauscher Lewatit TP 208 (H-Form) (Bayer AG); Reaktionstemperatur 50 °C; Ausgangskonzentration der wäßrigen Acroleinlösung 16.9 Gew.-%;
LHSV = 0,53 $Std^{-1}$.
Innerhalb einer Betriebszeit von 200 Stunden lag die Selektivität durchweg um 80 %.

**Beispiel 12**

Hydrierung einer gemäß Beispiel 11 erhaltenen wäßrigen Lösung von 3-Hydroxypropionaldehyd (HPA) an einem Festbettkontakt ($Ni/Al_2O_3/SiO_2$).

Die Reaktionslösung des Beispiels 11 wird bei 65 °C und unter vermindertem Druck von nicht-umgesetztem Acrolein und gleichzeitig einem Teil des Wassers befreit. In den Hydrierreaktor üblicher Bauart werden 750 ml einer 1,43 Mol HPA enthaltenden wäßrigen Lösung mit einem pH-Wert von 5 eingebracht und über 140 g Katalysator umgewälzt. Bei 55 °C wird bei einem $H_2$-Druck von 150 bar, der während der Hydrierung auf 105 bar absinkt, 4 Stunden hydriert. Der HPA-Umsatz beträgt 100 % die Ausbeute an 1,3-Propandiol 81 % (quantitative Gaschromatographie). Die Aufarbeitung der Reaktionslösung erfolgt in an sich bekannter Weise destillativ.

Auch bei wiederholtem Einsatz des Hydrierkatalysators bleiben der HPA-Umsatz und die Ausbeute an 1,3-Propandiol im wesentlichen unverändert hoch.

**Beispiel 13**

Hydrierung einer gemäß Beispiel 11 erhaltenen wäßrigen Lösung von 3-Hydroxypropionaldehyd (HPA).

Die Reaktionslösung des Beispiel 11 wird über eine Destillationskolonne bei 500 mbar von nichtumgesetztem Acrolein befreit. Anschließend wird die erhaltene wäßrige HPA-Lösung an einem Dünnschichtverdampfer bei einer Manteltemperatur von 100 °C und einem Druck von 200 mbar weiter eingeengt. 500 g der so erhaltenen 19 gew.-%igen Lösung werden in einem 1000 ml-Autoklaven mit Begasungsrührer bei 135 bar Wasserstoff-Druck, bei einer Temperatur von 75 °C und einer Rührerdrehzahl von 1000 UpM in Gegenwart von 5,8 g Raney-Nickel bei pH 7 innerhalb 60 min hydriert. Der HPA-Umsatz beträgt 99.9 % und die Ausbeute an 1,3-Propandiol 81 %, bezogen auf eingesetztes Acrolein. Die Aufarbeitung der Reaktionslösung erfolgt in an sich bekannter Weise destillativ.

**Patentansprüche**

1. Verfahren zur Herstellung von 1,3-Propandiol durch Hydratisierung von Acrolein in Gegenwart eines Ionenaustauschers unter Bildung von 3-Hydroxypropionaldehyd, wobei Acrolein und Wasser im Gewichtsverhältnis von 1:2 bis 1:20 bei 30 bis 120 °C und einem Druck im Bereich von 1 bis 20 bar umgesetzt werden, Abtrennung des Ionenaustauschers und, soweit vorhanden, des nicht umgesetzten Acroleins aus dem Reaktionsgemisch und anschließende katalytische Hydrierung des 3-Hydroxypropionaldehyds unter an sich bekannten Bedingungen in flüssiger oder gasförmiger Phase unter Verwendung üblicher

Hydrierkatalysatoren,
dadurch gekennzeichnet,
daß man chelatbildende Ionenaustauscher verwendet, welche an die Polymermatrix eines Polymerisationsharzes gebundene Ankergruppen der allgemeinen Formel

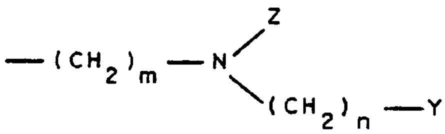

enthalten, worin bedeuten

| | |
|---|---|
| Z : | H, $C_1$- bis $C_6$-Alkyl, $-CH_2-CH(CH_3)-Y'$ oder $-(CH_2)_o-Y'$; |
| Y und Y' : | gleich oder verschieden; |
| | -COOH, -OH, Pyridyl oder $-P(O)(CH_2OH)OH$, wobei die sauren funktionellen Gruppen teilweise in Form ihrer Alkali-, Erdalkali- oder Erdmetallsalze vorliegen können. |
| m : | 0, 1, 2 oder 3 |
| n : | 1, 2 oder 3 für Y = -COOH, Pyridyl oder $-P(O)(CH_2OH)OH$; |
| | 2 oder 3 für Y = -OH |
| o : | 1, 2 oder 3 für Y' = -COOH, Pyridyl oder $-P(O)(CH_2OH)OH$; |
| | 0, 2 oder 3 für Y' = -OH . |

2. Verfahren nach Anspruch 1,
dadurch gekennzeichnet,
daß man chelatbildende Ionenaustauscher verwendet, welche Methyleniminodiessigsäure- oder Methyleniminodipropionsäure-Ankergruppen aufweisen.

3. Verfahren nach Anspruch 2,
dadurch gekennzeichnet,
daß ein Teil der Carboxylgruppen, vorzugsweise bis zu einem Drittel der Totalkapazität, in Form von Alkali-, Erdalkali- oder Erdmetallcarboxylaten vorliegt.

4. Verfahren nach Anspruch 1,
dadurch gekennzeichnet,
daß man chelatbildende Ionenaustauscher mit Ankergruppen der Formel

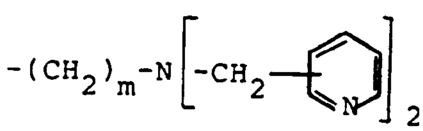

oder

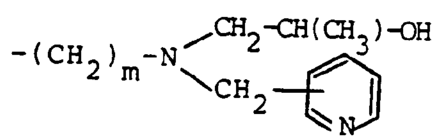

verwendet, worin m 0, 1, 2 oder 3, vorzugsweise 1 bedeutet.

5. Verfahren nach einem der Ansprüche 1 bis 3,
dadurch gekennzeichnet,
daß man in der Hydratisierungsstufe Acrolein und Wasser im Gewichtsverhältnis von 1:3 bis 1:10 einsetzt.

6. Verfahren nach einem oder mehreren der Ansprüche 1 bis 5,
dadurch gekennzeichnet,
daß man die Hydratisierung bei 40 bis 90 °C durchführt.

7. Verfahren nach einem oder mehreren der Ansprüche 1 bis 6,
dadurch gekennzeichnet,
daß man die Hydratisierung bis zu einem Acrolein-Umsatz von 30 bis 90 % betreibt, anschließend den Ionenaustauscher abtrennt und dann nichtumgesetztes Acrolein zwecks Rückführung in die Hydratisierungsstufe aus dem wäßrigen Reaktionsgemisch abdestilliert.

**8.** Verfahren nach einem oder mehreren der Ansprüche 1 bis 7,
dadurch gekennzeichnet,
daß man den Ionenaustauscher in einem Festbettreaktor anordnet und das Volumen des Festbettes sowie den Durchfluß und die Reaktionstemperatur so aufeinander abstimmt, daß durch einmalige Passage des Reaktionsgemisches durch den Reaktor der gewünschte Acrolein-Umsatz erreicht wird.

**9.** Verfahren nach einem oder mehreren der Ansprüche 1 bis 8,
dadurch gekennzeichnet,
daß man die katalytische Hydrierung des 3-Hydroxypropionaldehyds in wäßriger Lösung unter Druck bei 20 bis 250 bar im Temperaturbereich von 40 bis 140 °C und im pH-Bereich von 3,0 bis 8,5 durchführt.

**10.** Verfahren nach einem oder mehreren der Ansprüche 1 bis 9,
dadurch gekennzeichnet,
daß man die Hydrierung von 3-Hydroxypropionaldehyd in Gegenwart von nickel- oder edelmetallhaltigen Suspensions- oder Festbettkatalysatoren durchführt.

## Claims

**1.** Process for the preparation of 1,3-propanediol by hydration of acrolein in the presence of an ion exchanger with formation of 3-hydroxypropionaldehyde, wherein acrolein and water are reacted in a ratio by weight of from 1:2 to 1:20 at from 30 to 120°C and at a pressure within the range 1 to 20 bar, separation of the ion exchanger and of any unreacted acrolein present from the reaction mixture, and subsequent catalytic hydrogenation of the 3-hydroxypropionaldehyde in the liquid or gas phase under conditions which are known per se and using conventional hydrogenation catalysts, characterized in that chelate-forming ion exchangers are used which contain anchoring groups of the general formula

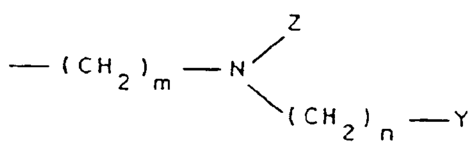

bonded to the polymer matrix of a polymerisation resin, wherein

| | |
|---|---|
| Z | : denotes H, $C_1$- to $C_6$-alkyl, $-CH_2-CH(CH_3)$ -Y′ or $-(CH_2)_o$-Y′; |
| X and Y′ | : are identical or different and denote -COOH, -OH, pyridyl or $-P(O)(CH_2OH)OH$, wherein some of the acid functional groups may be present in the form of their alkali metal, alkaline earth metal or earth metal salts; |
| m | : denotes 0, 1, 2 or 3 |
| n | : denotes 1, 2 or 3 if Y = -COOH, pyridyl or $-P(O)(CH_2OH)OH$; and 2 or 3 if Y = -OH; |
| o | : denotes 1, 2 or 3 if Y′ = -COOH, pyridyl or $-P(O)(CH_2OH)OH$; and 0, 2 or 3 if Y′ = -OH. |

**2.** Process according to Claim 1, characterized in that chelate-forming ion exchangers are used which have methyleneiminodiacetic acid anchoring groups or methyleneiminodipropionic acid anchoring groups.

**3.** Process according to Claim 2, characterized in that a portion of the carboxyl groups, preferably up to one third of the total capacity, is present in the form of alkali metal, alkaline earth metal or earth metal carboxylates.

**4.** Process according to Claim 1, characterized in that chelate-forming ion exchangers having anchoring groups of the formula

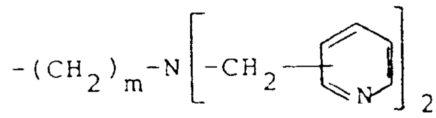

or

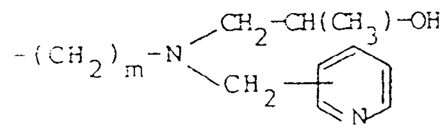

are used, wherein m denotes 0, 1, 2 or 3, and preferably 1.

5. Process according to one of Claims 1 to 3, characterized in that acrolein and water are utilized in a ratio by weight of from 1:3 to 1:10 in the hydration stage.

6. Process according to one or more of Claims 1 to 5, characterized in that hydration is performed at from 40 to 90°C.

7. Process according to one or more of Claims 1 to 6, characterized in that hydration is pursued until from 30 to 90% acrolein conversion is achieved, the ion exchanger is subsequently separated and unreacted acrolein is then distilled out of the aqueous reaction mixture in order to be recycled into the hydration stage.

8. Process according to one or more of Claims 1 to 7, characterized in that the ion exchanger is disposed in a fixed bed reactor and the volume of the fixed bed and flow rate and reaction temperature are adjusted to one another in such a manner that the desired acrolein conversion is achieved in a single passage of the reaction mixture through the reactor.

9. Process according to one or more of Claims 1 to 8, characterized in that the catalytic hydrogenation of the 3-hydroxypropionaldehyde is performed in an aqueous solution at a pressure of from 20 to 250 bar within the temperature range 40 to 140°C and within the pH range 3.0 to 8.5.

10. Process according to one or more of Claims 1 to 9, characterized in that the hydrogenation of 3-hydroxypropionaldehyde is performed in the presence of suspension or fixed bed catalysts containing nickel or noble metal.

## Revendications

1. Procédé d'obtention du 1,3-propanediol par hydratation de l'acroléine en présence d'un échangeur d'ions avec formation de l'aldéhyde 3-hydroxypropionique , procédé dans lequel l'acroléine et l'eau sont mis à réagir dans un rapport pondéral allant de 1 : 2 à 1 : 20, entre 30 et 120°C, et à une pression dans la zone de 1 à 20 bars, avec séparation de l'échangeur d'ions et -pour autant qu'elle soit présente- de l'acroléine qui n'a pas réagi du mélange réactionnel et hdyrogénation catalytique corrélative de l'aldéhyde 3-hydroxypropionique dans des conditions connues en soi, en phase liquide ou gazeuse, en utilisant des catalyseurs d'hydrogénation habituels, procédé caractérisé en ce que l'on utilise des échangeurs d'ions formant des chélates, qui contiennent des groupes d'ancrage liés à la matrice polymérique d'une résine de polymérisation, de formule générale :

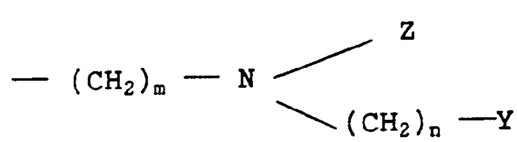

dans laquelle :
Z signifie H, alcoyl en $C_1$ à $C_6$, - $CH_2$- CH ($CH_3$) - Y′, ou $(CH_2)_o$ - Y ;
Y et Y′, identiques ou différents, signifient - COOH, - OH, pyridyle, ou - P(O)($CH_2$ OH) OH, les groupes fonctionnels acides pouvant se présenter partiellement sous forme de leurs sels de métal alcalin, de métal alcalino-terreux ou de métal terreux,
m signifie 0, 1, 2 ou 3
n signifie 1, 2 ou 3 pour Y = - COOH, pyridyle ou - P(O)($CH_2$OH) OH
ou 2 ou 3 pour Y = - OH
o signifie 1, 2 ou 3 pour Y′ = - COOH, pyridyle ou - P(O) ($CH_2$OH) (OH)
ou o, 2 ou 3 pour Y′ = - OH.

2. Procédé selon la revendication 1, caractérisé en ce que l'on utilise des échangeurs d'ions formant des chélates qui possèdent des groupes d'ancrage du type acide méthylène-imino-diacétique ou acide méthylène iminodipropionique.

3. Procédé selon la revendication 2, caractérisé en ce qu'une partie des groupes carboxyliques -de préférence jusqu'à un tiers de leur capacité totale- se présente sous la forme de carboxylates alcalins, alcalino-terreux ou de carboxylates de métaux terreux.

4. Procédé selon la revendication 1, caractérisé en ce que l'on utilise des échangeurs d'ions formant des chélates avec des groupes d'ancrage de formule :

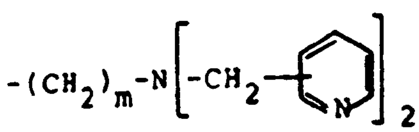

ou

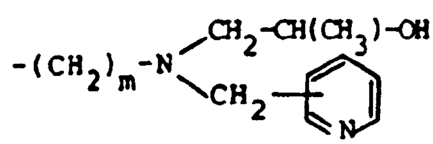

dans laquelle m signifie 0, 1, 2 ou 3 et de préférence 1.

5. Procédé selon l'une quelconques des revendications 1 à 3, caractérisé en ce que l'on met en oeuvre dans l'étape d'hydratation l'acroléine et l'eau dans un rapport pondéral allant de 1 : 3 à 1 : 10.

6. Procédé selon une ou plusieurs des revendications 1 à 5, caractérisé en ce que l'hydratation est effectuée entre 40 et 90° C.

7. Procédé selon une ou plusieurs des revendications 1 à 6, caractérisé en ce que l'on poursuit l'hydratation jusqu'à un taux de conversion de l'acroléine de 30 à 90 %, ensuite on sépare l'échangeur d'ions, puis on sépare par distillation l'acroléine qui n'a pas réagi, aux fins de recyclage dans l'étape d'hydratation, du mélange réactionnel aqueux.

8. Procédé selon une ou plusieurs revendications 1 à 7, caractérisé en ce que l'on dispose l'échangeur d'ions dans un réacteur à lit solide et que l'on met en accord le volume du lit solide ainsi que le débit et la température de réaction l'un par rapport à l'autre, de sorte que par passage une fois du mélange réactionnel à travers le réacteur, le taux de conversion d'acroléine désiré soit atteint.

9. Procédé selon une ou plusieurs des revendications 1 à 8, caractérisé en ce que l'on effectue l'hydrogénation catalytique de l'aldéhyde 3-hydroxypropionique en solution aqueuse à une pression de 20 à 250 bars dans une zone de température de 40 à 140°C et dans une zone de pH de 3,0 à 8,5.

10. Procédé selon une ou plusieurs des revendications 1 à 9, caractérisé en ce que l'on effectue l'hydrogénation de l'aldéhyde 3-hydroxypropionique en présence de catalyseurs en suspension ou en lit solide contenant du nickel ou un métal noble.